# EUROPEAN PATENT APPLICATION

(11) **EP 4 270 445 A1**
(43) Date of publication of application: **01.11.2023**
(21) Application number: 23165018.5
(22) Date of filing: 29.03.2023
(51) Int. Cl.: H01J 61/33, A61L 2/00, A61L 2/10, A61L 9/20, H01J 61/35, H01J 61/40, H01J 65/04

(54) **AN ULTRAVIOLET LAMP TUBE**

(30) Priority: 28.04.2022 CN 202221017401 U
(71) Applicant: Langsim Optoelectronic Technologies (Guangdong) Limited, Nanhai, Foshan Guangdong (CN)
(72) Inventor: Li, Jiawei, XIAMEN, Fujian Province (CN); Li, Zhen, HANGZHOU, Zhejiang Province (CN)
(74) Representative: Díaz de Bustamante y Terminel, Isidro

(57) **Abstract**

This application discloses an ultraviolet lamp tube, comprising an inner tube 100 and an outer tube 200, the inner tube installed to penetrate through inside of the outer tube, an excitation chamber 300 formed between the inner tube and the outer tube; an inner electrode 400 installed in the inner tube, an outer electrode 500 installed on the outer surface of the outer tube; the outer tube comprising a curved surface part 210 and a flat surface part 220, wherein the curved surface part and the flat surface part are connected to each other, the curved surface part is an arc-shaped surface arranged coaxially with the inner tube, and the flat surface part is a plane; and wherein a first film layer 221 is arranged on the flat surface, and a second film layer 211 is arranged on the curved surface. The ultraviolet lamp tube of the application filters light wave by means of coating, which can ensure the monochromaticity of the light output of the light source, and at the same time, the ultraviolet lamp tube is designed in a cylindrical shape to effectively increase the luminous angle of the ultraviolet lamp tube, thus effectively improving the light efficiency of the ultraviolet lamp tube and greatly reducing production costs.

## Description

### Technical field

This utility model relates to the technical field of ultraviolet lamps, in particular to an ultraviolet lamp tube.

### Background Technology

Ultraviolet light sources are mostly used for medical purposes such as sterilization and disinfection, analysis purposes such as changes based on irradiated ultraviolet light, industrial purposes such as ultraviolet curing, cosmetic purposes such as ultraviolet tanning, and other purposes such as insect trapping, counterfeit currency identification, etc.

The existing UV lamp is filtered by a filter to filter out useless wavelength bands, but the structure still has the defects of small light-emitting angle and low light efficiency, as well as complex structures and high costs. To this end, we propose a new type of UV lamp tube to solve the above problems.

### Utility Model Content

In view of the defects of the existing technology, this utility model provides an ultraviolet lamp tube, which has the characteristics of large luminous angle, high light efficiency, simple structure and low cost.

The purpose of this utility model is realized by adopting following technical scheme:
An ultraviolet lamp tube, including an inner tube and an outer tube,
the inner tube penetrating through inside of the outer tube, an excitation chamber formed between the inner tube and the outer tube; an inner electrode arranged in the inner tube;
the outer tube including a curved surface part and a flat surface part, the curved surface part and the flat surface part connected to each other, wherein the curved surface part is an arc-shaped surface arranged coaxially with the inner tube, and the flat surface part is a plane; and wherein a first film layer is arranged on the flat surface, a second film layer is arranged on the curved surface, and the outer surface of the curved portion is provided with an outer electrode.

In one embodiment, the wavelengths of the wave bands that the first film layer and the second film layer can transmit are different.

In one embodiment, the first film layer is a filter film layer, and the filter film layer has a property of transmitting selected wavelengths.

In one embodiment, the filter film layer is a single-wavelength film layer, and the single-wavelength coating has a property of light unity; the single-wavelength coating can only transmit a single-wavelength waveband to the outside of the flat surface portion.

In one embodiment, the first film layer is plated on the outer surface of the flat surface portion.

In one embodiment, the second film layer is a reflective film layer, and the reflective film layer has a light reflection property and is used to reflect any wavelength band back to the excitation chamber.

In one embodiment, the reflective film layer is a reflective coating spray coated on the outer surface of the curved portion.

In one embodiment, the second film layer is a clutter wave film layer, and the clutter wave film layer can transmit the wavelength bands that cannot pass through the first film layer, and at the same time reflect the wavelength band that can pass through the first film layer back to the excitation chamber.

In one embodiment, the clutter wave film layer is coated on the outer surface of the curved portion.

Compared with the existing technology, the beneficial effects of the present utility model are:
The ultraviolet lamp tube of the utility model filters lights by means of film coating, which can ensure the monochromaticity of the wavelength of light emitted by the light source, and the volume of the ultraviolet lamp tube can be reduced by means of coating, which is convenient for use; by means of arranging the outer tube with a combination of the curved part and the flat part, and plating the second film layer and the first film layer with different wavelength transmitting properties on the curved part and the flat part, respectively, the light emission from the plane is achieved and the light output efficiency of the UV lamp is effectively improved; designing the ultraviolet lamp tube in a non-complete circular cylindrical shape can effectively increase the light-emitting angle of the UV lamp; by distinguishing the flat part and the curved part to locate the position of the light-emitting surface, it is easy to use, and during the coating process, the flat coating position is determined by locating the flat part position, which greatly improves the production efficiency, and also effectively improves the coating quality and reduces costs.

### Description of the Drawings

FIG. 1 is three-dimensional view of the ultraviolet lamp tube of the utility model;
FIG. 2 is a schematic cross-sectional view of an ultraviolet lamp tube of the utility model;
FIG. 3 is a perspective view of the internal structure of the ultraviolet lamp tube of the utility model.

In the figures: 100, inner tube; 200, outer tube; 210, curved surface part; 211, second film layer; 220, flat part; 221, first film layer; 300, excitation chamber; 400, inner electrode; 500, external electrode.

### Detailed Embodiments

Hereinafter, the present invention will be further described with reference to the accompanying drawings and specific embodiments. It should be noted that, under the premise of no conflicts, the embodiments or technical features described below can be arbitrarily combined to form new embodiments. Unless otherwise specified, the materials and equipment used in the embodiments can be purchased from the market. Examples of such embodiments are illustrated in the accompanying drawings, wherein the same or similar reference numerals refer to the same or similar elements or elements having the same or similar functions throughout. The embodiments described below with reference to the accompanying drawings are illustrative and only used to explain the present application and should not be construed as limiting the present application.

In the description of this application, it is to be understood that the terms "up", "down", "front", "rear", "vertical", "horizontal", "top", "bottom", "inner", "outside", etc., refer to the orientation or positional relationship based on the orientation or positional relationship shown in the accompanying drawings, which is only for the convenience of describing the present application and simplifying the description, rather than indicating or implying that the device or element referred to must have a specific orientation, or operated in a particular orientation, and is therefore not to be construed as a limitation of the present application. In the description of this application, "plurality" means two or more, unless it is precisely and specifically defined otherwise.

In the description of the present application, it should be noted that, unless otherwise expressly specified and defined, the terms "connect", "connected" and "joint" should be understood in a broad sense, for example, it can be a fixed connection, or can be a connection via an intermediary media, can be the internal connection of two elements or the interaction relationship between the two elements. For those of ordinary skill in the art, the specific meanings of the above terms in this application can be understood according to specific situations.

The terms "first", "second" and the like in the description and claims of the present application and the above drawings are used to distinguish similar objects and are not necessarily used to describe a specific order or sequence. Furthermore, the terms "comprising" and "having", and any variations thereof, are intended to cover non-exclusive inclusion, for example, a process, method, system, product or device comprising a series of steps or units is not necessarily limited to those expressly listed, rather, may include other steps or units not expressly listed or inherent to these processes, methods, products or devices.

### Example 1:

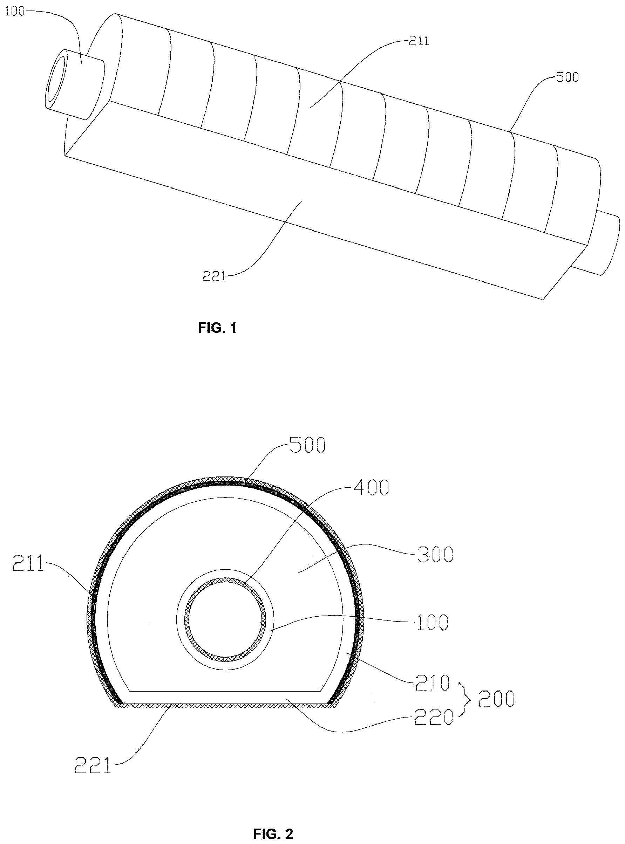

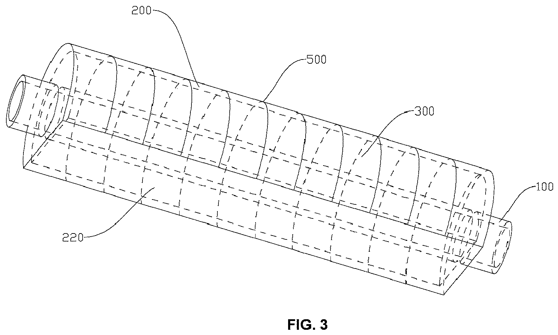

Referring to Fig. 1-3, a UV lamp tube includes an inner tube 100 and an outer tube 200, the inner tube 100 penetrates through inside the outer tube 200; an excitation chamber 300 is formed between the inner tube 100 and the outer tube 200; an inner electrode 400 is arranged in the inner tube 100. Specifically, the inner electrode 400 is arranged on the inner wall of the inner tube 100; an outer electrode 500 is arranged on the outer surface of the curved portion 210 of the outer tube 200.

Referring to the cross-sectional view of the UV lamp tube shown in Fig. 2, the outer tube 200 includes a curved surface portion 210 and a flat surface portion 220 connected to each other, the curved surface portion 210 is a circular arc-shaped surface coaxially arranged with the inner tube 100, and the flat surface portion 220 is a plane;

The curved portion 210 is plated with a second film layer 211, and the flat portion 220 is plated with a first film layer 221; the wavelengths that the first film layer 221 and the second film layer 211 can transmit are different.

The ultraviolet lamp tube in this embodiment filters lights by means of film coating, which can ensure the unity of the wavelength of light emitted by the light source, and the volume of the ultraviolet lamp can also be reduced by means of coating, which is convenient for use; by designing the outer tube 200 as a combination of curved surface 210 and flat part 220, and coating the curved part 210 and the flat part 220 respectively with a second film layer 211 and a first film layer 221, which can transmit different wavelengths, so as to achieve light emission from the plane and effectively improve the luminous efficiency of the UV lamp tube; the luminous angle of the UV lamp tube can be effectively increased by designing the UV lamp tube in a non-completely circular cylindrical shape; the position of the light emitting surface can be set by distinguishing the flat surface portion 220 and the curved surface portion 210, which is convenient for use, and during the coating process, by locating the position of the plane portion 220 to determine the plane coating position, the production efficiency can be greatly improved, the coating quality can be effectively improved, and the cost can be reduced.

### Example 2:

A UV lamp tube as shown in Fig. 1-3, in this example, on the basis of Example 1, the first film layer 221 is a filter film layer, and the filter film layer has a property of transmitting selected wavelengths; the second film layer 211 is a reflective film layer, and the reflective film layer has light reflection properties.
The first film 221 can selectively transmit a single wavelength band required, such as 207 nm, 222 nm, 250 nm, 308 nm, etc.; The second film layer 211 is a reflective film layer that can reflect any wavelength band back to the excitation chamber 300.

By setting the second film layer 211 as a reflective film layer, the wavelength band projected to the second film layer 211 is reflected back to the excitation chamber 300, and then projected to the first film layer 221 through the reflection of the curved surface portion 210, and the filter film layer with a wavelength selection property transmits the desired wavelength band and filters out useless clutter waves, and also enhances the light efficiency of the UV lamp.

### Example 3:

A UV lamp tube as shown in Fig. 1-3, this example is based on Example 1. The first film layer 221 is a light filter coating, and the light filter coating has the property of transmitting selected wavelengths; the second film layer 211 is a clutter wave film layer, the clutter wave film layer can transmit the wavelength bands that cannot pass through the first film layer 221, and at the same time reflect the wavelength band that can pass through the first film layer 221 back to the excitation chamber 300, which is further projected to the first film layer 221 through reflection and emitted out.

The first film layer 221 can transmit useful single wavelength band, such as 207 nm, 222 nm, 250 nm, 308 nm, etc., while the second film layer 211 can project the wavelength bands that cannot pass through the first film layer 221, and at the same time reflect the single wavelength band that can pass through the first film layer 221 back to the excitation chamber 300.

The useful single wavelength band is reflected back to the excitation chamber 300 through the clutter wave film layer, and projected to the first film layer 221 through reflection and emitted out, which effectively improves the light efficiency of the ultraviolet lamp; the wavelength bands that cannot pass through the film layer 221 are emitted out by the second film layer 211.

In some other embodiments, the first film layer 221 is coated on the outer surface of the flat portion 220, which is beneficial to increase the volume of the excitation chamber 300 and improve the light efficiency. The second film layer 211 is a reflective coating spray coated on the outer surface of the curved portion 210, or a clutter wave coating coated on the outer surface of the curved portion 210, which is also beneficial to increase the volume of the excitation chamber 300 and improve the light efficiency. Further, by means of coating, while improving the light efficiency, it also makes the structure more simple and compact and improves the practicability of the ultraviolet lamp tube.

This utility model adopts the method of film coating for filtering light, which can ensure the monochromaticity of the light output of the light source. Secondly, the method of film coating can enhance the output power of ultraviolet light, improve the photoelectric conversion efficiency, and at the same time, the luminous angle of the ultraviolet lamp can be effectively increased by the area ratio of the first film layer 221 and the second film layer 21, thus effectively improving the light efficiency of the ultraviolet lamp tube.

While only certain components and embodiments of the present application have been illustrated and described, many modifications and changes can occur to those skilled in the art without actually departing from the scope and spirit of the claims (e.g., variations in size, dimensions, configuration, shape and proportions, mounting arrangements, material usage, color, orientation, etc. of individual elements).

Finally, it should be noted that the above-mentioned embodiments are only the preferred embodiments of the present invention, and the scope of protection of the present invention cannot be limited by this. Any non-essential changes and substitutions by those skilled in the art on the basis of the present invention belong to the scope of protection requested by the present invention.

## Claims

1. An ultraviolet lamp tube, **characterized by**, comprising an inner tube and an outer tube, the inner tube installed to penetrate through inside of the outer tube, wherein an excitation chamber is formed between the inner tube and the outer tube; an inner electrode arranged in the inner tube; wherein the outer tube comprises a curved surface part and a flat surface part, the curved surface part and the flat surface part connected to each other, wherein the curved surface part is an arc-shaped surface arranged coaxially with the inner tube, and the flat surface part is a plane; wherein a first film layer is arranged on the flat surface, and a second film layer is arranged on the curved surface, and wherein an outer electrode is installed on the curved portion of the outer surface.

2. The ultraviolet lamp tube according to claim 1, **characterized by**, the wavelengths of the wave bands that the first film layer and the second film layer can transmit are different.

3. The ultraviolet lamp tube according to claim 2, **characterized by**, the first film layer is a light filtering film layer, and the light filtering film layer possesses a property of transmitting selected wavelengths.

4. The ultraviolet lamp tube according to claim 3, **characterized by**, the light filtering film layer is a single-wavelength film layer, and the single-wavelength coating possessing a property of light unity; the single-wavelength coating can only transmit a single-wavelength waveband to the outside of the flat surface part.

5. The ultraviolet lamp tube according to claim 1, **characterized by**, the first film layer is coated on the outer surface of the flat portion.

6. The ultraviolet lamp according to claim 3, **characterized by**, the second film layer is a reflective film layer, and the reflective film layer has the property of light reflection, used to reflect any wavelength band back to the excitation chamber.

7. The ultraviolet lamp tube according to claim 6, **characterized by**, the reflective film layer is a reflective coating spray coated on the outer surface of the curved portion.

8. The ultraviolet lamp according to claim 3, **characterized by**, the second film layer is a clutter wave film layer, and the clutter wave film layer can transmit the wavelength band that cannot pass through the first film layer, and at the same time, can reflect the wavelength band that can pass through the first film layer back to the excitation chamber.

9. The ultraviolet lamp according to claim 8, **characterized by**, the clutter wave film is coated on the outer surface of the curved portion.
